# EUROPÄISCHE PATENTSCHRIFT

(11) **EP 0 880 339 B1**
(45) Veröffentlichungstag und Bekanntmachung des Hinweises auf die Patenterteilung: **31.07.2002**
(21) Anmeldenummer: 97903272.9
(22) Anmeldetag: 13.02.1997
(51) Int. Cl.: A61B 5/00

(54) **GEHIRN-SAUERSTOFFPARTIALDRUCK-MESSVORRICHTUNG**
BRAIN OXYGEN PARTIAL PRESSURE MEASURING DEVICE
DISPOSITIF DE MESURE DE LA PRESSION PARTIELLE D'OXYGENE DU CERVEAU

(30) Priorität: 16.02.1996 DE 19605739
(43) Veröffentlichungstag der Anmeldung: 02.12.1998
(73) Patentinhaber: GMS, Gesellschaft für medizinische Sondentechnik mbH, D-2301 Mielkendorf (DE)
(72) Erfinder: Fleckenstein, Wolfgang, 24247 Mielkendorf (DE)
(74) Vertreter: Schaefer, Konrad, Dipl.-Phys.
(86) Internationale Anmeldenummer: EP9700669
(87) Internationale Veröffentlichungsnummer: WO9729681

(56) Entgegenhaltungen:
- WO-A-91/09302
- WO-A-93/06776
- US-A- 4 705 617
- US-A- 4 950 378
- PATENT ABSTRACTS OF JAPAN vol. 9, no. 239 (P-391), 25.September 1985 & JP 60 093343 A (NIHON DENCHI KK), 25.Mai 1985,

## Beschreibung

Die Erfindung betrifft eine Meßvorrichtung der im Oberbegriff des Anspruches 1 genannten Art.

Aus
DE-Z Acta Neurochir
1993 (Suppl) 59, Seiten 50 - 57
auf Seite 51 unter der Überschrift "Brain Tissue pO₂-Measurements", sowie aus
DE-Z:Medizintechnik, 110,
Heft 2, 1990, Seiten 44 - 53
(Sonderdruck)
und
Acta Neurochirurgica, 1993 (Suppl.),
59, Seiten 50 - 57
ist es bekannt, mit einer dünnen, flexiblen Kathetersonde vom Clark-Typ direkt im Gehirngewebe zu messen.

Aus
Z Biotelemetry Patient Monitoring 6
1979, Seiten 16 - 31
und
DE-Z Proceedings Dtsch. Ges. Biomed. Techn.
(1973) pp 29, 30
"Sauerstoffsensoren für in vivo-Messungen"
W. Mindt
sind sehr dünne und hochflexible schlauchförmige Katheter, die eine pO₂-Sonde vom Clark-Typ ausbilden, erläutert, die für diesen Zweck geeignet ist.

Solche Sonden können bei geeigneter Verlegetechnik, wie in der nachveröffentlichten Patentanmeldung
DE 195 02 183.5
beschrieben, frei im Gehirngewebe verlegt werden. Aufgrund ihres geringen Durchmessers und ihrer hohen Flexibilität sind solche Sonden zur Langzeitmessung im Gehirngewebe und sonstigen empfindlichen Geweben des Körpers geeignet. Sie können den Bewegungen des Gewebes (Puls, Atmung) folgen, ohne dieses zu zerstören und üben nur geringe Druckeffekte auf das umgebende Gewebe aus, so daß die Sauerstoffversorgung nicht in den Meßwert verfälschender Weise beeinflußt wird.

Andererseits haben solche extrem dünnen Sonden aufgrund ihrer geringen Abmessungen Nachteile hinsichtlich der technischen Durchführbarkeit von Lanzeitmessungen.

Eine Sonde der gattungsgemäßen Art hat typischerweise einen Innendurchmesser von wenigen Zehntel Millimetern bei einer Länge von einigen dm. Daraus ergibt sich ein mit Elektrolyt füllbarer Innenraum in der Größenordnung weniger mm³, wovon aber nur ein sehr kleiner Teil im Bereich der sehr kleinen Kathodenoberfläche nutzbar ist. Ein solch geringer Elektrolytvorrat ist für pO₂-Sonden des Clark-Typs ungewöhnlich klein und für Langzeitmessungen nachteilig.

pO₂-Sonden des Clark-Typs setzen einen Elektrolytraum über eine Sauerstoffdiffusionsmembran dem umgebenden Sauerstoff aus. Der Sauerstoff diffundiert durch die Membran gegen den Diffusionswiderstand. Die in den Elektrolyt pro Zeiteinheit eintretende Sauerstoffmenge hängt vom Sauerstoffpartialdruck in der Umgebung ab. Mit zwei Elektroden, einer aus geeignetem Edelmetall bestehenden Kathode und einer üblicherweise aus Silber bestehenden Anode, gegebenenfalls mit Silberchloridoberfläche, wird der eintretende Sauerstoff reduziert. Der resultierende Stromfluß (Meßstrom) hängt eindeutig vom eindiffundierenden Sauerstoffstrom, also dem umgebenden Sauerstoffpartialdruck ab. An die Elektroden ist dazu eine geeignete Polarographiespannung anzulegen.

Als Elektrolyt werden wässrige Salzlösungen, üblicherweise Natriumchlorid, verwendet. Bei der Reduktion des Sauerstoffes entsteht Hydroxyd. Silber von der Anode geht als Silbersalz in Lösung. Silber ist an der Anode zwar in ausreichendem Vorrat vorhanden. Der sehr kleine Elektrolytvorrat der galtungsgemäßen Sonden wird jedoch relativ schnell verbraucht. Es ändern sich die elektrochemischen Gleichgewichte, so daß sich der Zusammenhang zwischen dem Meßstrom und dem einströmenden Sauerstoffstrom ändert. Die Messung muß dann abgebrochen und die Sonde ersetzt werden. Eine Neubefüllung mit Elektrolyt vor Ort, also im Gewebe, ist ausgeschlossen.

In
DE-Z Laborpraxis - Juli/August 1984, pp 736 - 739
"Ein neues Sauerstoff-Meßsystem"
Klaus Rommel
ist für Sonden dieser Art das Problem des Elektrolytverbrauchs beschrieben und zur Lösung dieses Problems eine 3-Elektrodenanordnung vorgeschlagen, die jedoch für im Gehirn verwendbare Sonden aus Gründen des erforderlichen geringen Durchmessers und der hohen Flexibilität nicht geeignet ist und mit der auch nur der Elektrolytverbrauch erkannt, nicht aber behoben werden kann.

Aus
GB-Z:Medical and Biological Engineering
and Computing, 30, 1992, Seiten 121 - 122
ist eine medizinische pO₂-Sonde bekannt, die zwar grundsätzlich nach dem Clark-Prinzip arbeitet, jedoch nicht die für dieses Prinzip typische, nur für Gase durchlässige Membran aufweist, sondern eine Membran aus Polyurethan. Dieses Material ist wasserdurchlässig, was bei dieser Konstruktion auch notwendig ist, um das unter der Membran getrocknet vorliegende Elektrolytsalz zu wässern. Da die Membran aber auch ionendurchlässig ist, ist die Sonde abhängig vom pH der Umgebung und kann nur bei konstantem UmgebungspH genau messen. Es ist daher vorgesehen, die Messungen in einer phosphatgepufferten Lösung durchzuführen. Für medizinische Anwendungen ist diese Sonde schlecht geeignet, da sie vom schwankenden pH der Umgebung abhängt und auch Salze ihres getrockneten Elektrolytvorrates nach außen abgeben kann, also zu Gewebeschädigungen führen kann.

Aus
US 49 50 378
ist eine zur Znckerbestimmung in biologischer Umgebung dienende Sonde bekannt, die auf einer eigentlichen Sonde, die nach dem Clark-Prinzip arbeiten kann, ein Enzym vorsicht, das Zucker in H₂O₂ umsetzt. Es kommt bei dieser Sonde zu Vergiftungen der Kathodenoberfläche, die durch zeitweiligen Betrieb mit umgekehrter Polarität beseitigt werden. Probleme mit dem Elektrolytvorrat bestehen bei dieser Sonde nicht.

Aus der WO-A-91/09302 ist eine gattungsgemäße Meßvorrichtung bekannt.

Bei dieser Konstruktion kann nach einer Betriebszeit, über die so wenig Elektrolyt verbraucht wird, daß die Meßparameter noch stabil bleiben, auf Regenerierbetrieb umgeschaltet werden. Es wird dann mit einem in zum Meßstrom umgekehrter Richtung fließenden Regenerierstrom unter Umkehrung der beim Messen ablaufenden chemischen Reaktionen der Elektrolyt regeneriert.

Diese bekannte Konstruktion ist daher für lange Meßzeiten geeignet, wobei allerdings bei angestrebtem automatischem Betrieb das Meßgerät stets angeschlossen werden muß, um den Elektrolytverbrauch und daraus den erforderlichen Zeitpunkt zum Umschalten auf Regenerationsbetrieb berechnen zu können. Wird eine solche Sonde im rauhen Krankenhausalltag verwendet, so ginge bei jedem aus Betriebsgründen erforderlichen Anschluß an ein anderes Meßgerät die Vorgeschichte verloren, so daß der Regenerierbetrieb nicht mehr gesteuert werden kann.

Die Aufgabe der vorliegenden Erfindung besteht darin, Störungen des Regenerierbetriebes bei Wechsel des Meßgerätes zu verhindern.

Diese Aufgabe wird erfindungsgemäß mit den Merkmalen des Anspruches 1 gelöst.

Der der Sonde zugeordnete Datenträger kann neben sonstigen Sondenparametern, die z.B. Angaben über die Herstellung, Seriennummern, Eichdaten, Patientendaten u. dgl. speichern und kann insbesondere auch die Meßzeit und den Verlauf des Meßstromes während der vorangegangenen Meßzeit speichern. Wird mitten in einer Messung die Sonde vom Meßgerät abgekoppelt und an ein anderes Meßgerät angekoppelt, so kann dieses mit der Messung bis zum zulässigen Elektrolytverbrauch fortfahren und dann erst auf Regenerieren umschalten.

Dies ist in vielen Fällen von Vorteil, wenn die Sonde vom Meßgerät abgekoppelt werden muß, beispielsweise beim Waschen des Patienten und insbesondere beim Verlegen in ein anderes Bett. Die Meßvorrichtung muß dann nicht mitgenommen werden, sondern es kann die am neuen Bett vorhandene Meßvorrichtung verwendet werden.

Vorteilhaft sind dabei die Merkmale des Anspruches 2 vorgesehen. Diese Salze, z.B. in Form von Natrium- oder Kaliumsalzen, haben gegenüber den üblicherweise verwendeten Halogenidsalzen den Vorteil, daß die beim Messen entstehenden Silbersalze wesentlich besser löslich sind als Silberhalogenidsalze. Daher gelingt im Regenerierbetrieb die nahezu vollständige Wiederabscheidung des Silbers als Voraussetzung für eine entsprechend hohe Elektrolytregenerierung. Dabei wird in an sich bekannter Weise die Pufferwirkung der Salz/Säure-Mischung verwendet, um ,den durch die Reduktion des Sauerstoffs bewirkten Anstieg des pH; der die Meßparameter der Sonde beeinflußt, in sicheren Grenzen zu halten.

Vorteilhaft sind die Merkmale des Anspruches 3 vorgesehen. Bei Clark-Sonden mit größerem Elektrolytvorrat und kurzen Meßzeiten werden Anoden mit Silbersalz- oder -oxidoberfläche (Elektrode zweiter Art) bevorzugt. Bei diesen hängen die Meßparameter aber ab von der Konzentration des Anions des Elektrolyten. Bei der gattungsgemäßen Sonde mit sehr kleinem Elektrolytvorrat und langen Meßzeiten ist eine reine Silberanode (Elektrode erster Art) vorzuziehen, bei der diese Abhängigkeit nicht besteht, bei der also über längere Zeit mit stabilen Sondenparametern gemessen werden kann.

Vorteilhaft sind die Merkmale des Anspruches 4 vorgesehen. Es ist unmöglich, von außen die Elektrolytverhältnisse, z.B., auf chemischem Wege oder auf elektrischem Wege zu bestimmen, also den Zeitpunkt, zu dem der Elektrolyt so weit verbraucht ist, daß auf Reversierbetrieb umgeschaltet werden muß. Dies wäre nur mit gesonderten Meßeinrichtungen möglich, die bei gattungsgemäßen Sonden schon aus Platzgründen ausscheiden. Wird die Meßdauer und der Meßstrom bestimmt, so läßt sich daraus der Elektrolytverbrauch berechnen, beispielsweise aus dem Integral des Stromes über die Zeit. Es ergibt sich ein genaues Maß für den tatsächlichen Elektrolytverbrauch, und es kann exakt angegeben werden, wann aufgrund zu starken Elektrolytverbrauches die Sonde den vorgegebenen Parameterbereich verläßt und regeneriert werden muß. Die Meßvorrichtung kann also automatisch umschalten mit optimal langen Meßzeiten zwischen den Regenerierpausen.

In der Zeichnung ist die Erfindung beispielsweise und schematisch dargestellt. Es zeigen:
- Fig. 1: den teilgeschnittenen Kopf eines Patienten mit einer erfindungsgemäßen Meßvorrichtung mit Sonde und Meßgerät,
- Fig. 2: die Sonde der Meßvorrichtung der Fig. 1 im Axialschnitt und
- Fig. 3: ein stark schematisiertes Prinzipschaltbild des Meßgerätes der Fig. 1.

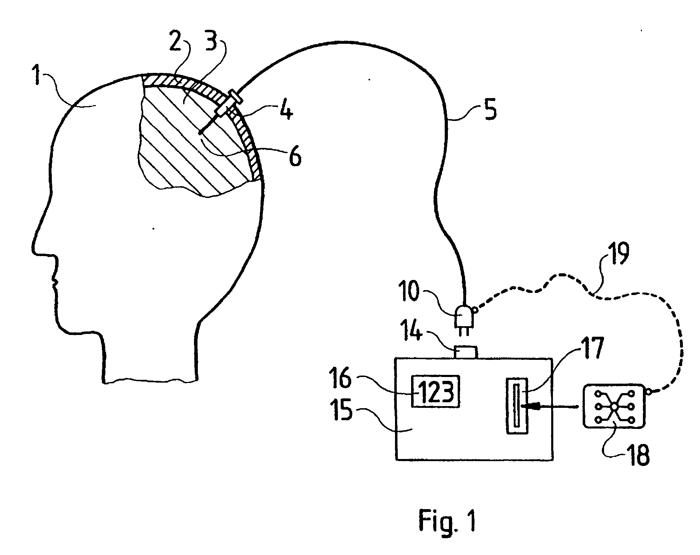

Fig. 1 zeigt den Kopf 1 eines Patienten, teilweise geschnitten mit Schädeldecke 2 und Gehimgewebe 3.

In einer Bohrung in der Schädeldecke 2 ist ein Einführungsstück 4 befestigt, z.B. eingeschraubt. Durch dieses ist eine Sonde 5 bis zum Meßort 6 in das Gehirngewebe 3 eingeiunrt. Das Einführungsstück 4 kann zum Einführen mehrerer Sonden ausgebildet sein und beispielsweise noch eine zusätzliche, nicht dargestellte Drucksonde aufnehmen, die den Innendruck des Gehirns bestimmt.

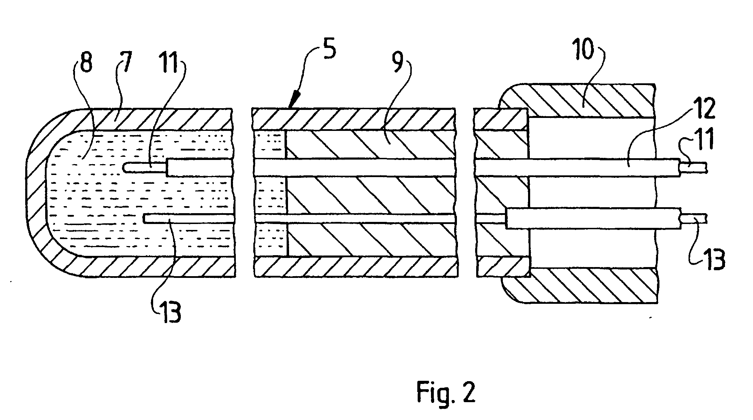

Die dargestellte Sonde 5 dient zur Bestimmung des Sauerstoffpartialdruckes im Gehirngewebe 3 am Meßort 6. Sie ist im Schnitt in Fig. 2 dargestellt.

Die Sonde 5 ist als pO₂-Sonde nach dein Clark-Prinzip ausgebildet. Sie weist einen umschließenden Schlauch 7 auf, der die Sauerstoffdiffusionsmembran ausbildet und der beispielsweise aus Polyethylen besteht. Der Schlauch weist beispielsweise einen Außendurchmesser von 0,6 mm und eine Wandstärke von 0,1 mm auf. Der vorn liegende Meßbereich der Sonde 5 ist uber eine Länge von mehreren cm mit Elektrolyt 8 gefüllt, vorzugsweise eine wässrige Lösung von Natriumazetat, Natriumphosphat, Essigsäure und Natriumdihydrogenphosphat zur Bildung eines großen Pufferbereiches, der den pH des Elektrolyten langfristig konstant halten.

Über die übrige Länge von z.B. 200 mm ist der Schlauch 7 mit Vergußmaterial 9 verschlossen. An seinem äußeren Ende ist ein Stecker 10 befestigt. Vom Stecker 10 kommend, verlaufen bis in den Elektrolytraum eine Kathode 11, z.B. aus Platin oder Gold, die im dargestellten Spitzenbereich blank liegt und über die übrige Länge mit einer Isolierung 12 versehen ist, sowie eine aus Silber bestehende Anode 13.

Alternativ kann auch der Schlauch beispielsweise über seine gesamte Länge mit Elektrolyt gefüllt sein, wobei nur ein kurzer Endbereich mit Vergußmaterial 9 verschlossen ist. Es ergeben sich dabei im wesentlichen dieselben Verhältnisse wie bei einem sehr kleinen Elektrolytraum, da der elektrochemische Prozeß nur im sehr engen Umgebungsbereich der Kathode abläuft. Weiter entfernte Bereiche eines langgestreckten größeren Elektrolytraumes bleiben aufgrund der langen Diffusionswege unbeteiligt.

Die Sonde 5 ist mit ihrem Stecker 10 an eine Steckkupplung 14 eines Meßgerätes 15 ankoppelbar. Das Meßgerät 15 weist, wie Fig. 1 zeigt, ein Anzeigedisplay 16 auf sowie ein Lesegerät 17, in dessen Einsteckschlitz ein Datenträger 18 einsteckbar ist, der im dargestellten vereinfachten Ausführungsbeispiel mit einer Sicherungskette 19 unverlierbar mit dem Stecker 10 der Sonde 5 verbunden ist.

Als Datenträger 18 kann jeder beliebige schreib- und lesbare handelsübliche Datenträger verwendet werden, wie er heutzutage z.B. als Scheckkarte oder Telefonkarte gebräuchlich ist. In Fig. 1 ist ein Datenträger mit Speicherchip und Kontaktpunkten dargestellt. Der Datenträger 18 kann auch mit einer Magnetoberfläche oder mit einer magneto-optisch beschreibbaren Oberfläche versehen sein. Das Lesegerät 17, das zum Beschreiben und zum Lesen des Datenträgers 18 mit Daten dient, ist passend zur Art des Datenträgers vorzusehen. Im einfachsten Beispiel könnte das Lesegerät 17 auch ein Diskettenlesegerät und der Datenträger 18 eine entsprechende Diskette sein, z.B. eine 3,5"-Diskette.

Der Datenträger kann anders als im dargestellten Ausführungsbeispiel auch auf sonstige Weise unverlierbar mit der Sonde 5 verbunden sein. Beispielsweise kann er in den Stecker 10 integriert sein, wobei die Steckkupplung 14 als Schreib/Lesegerät auszubilden ist.

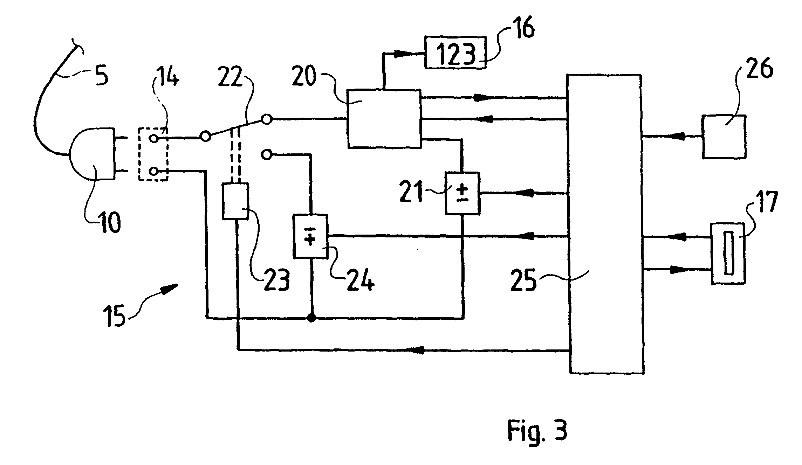

Das elektronische Innenleben des Meßgerätes 15 ist in Fig. 3 stark schematisiert als Blockschaltbild dargestellt.

Ist der Stecker 10 in die Kupplung 14 gesteckt und der in Fig. 3 nicht dargestellte Datenträger 18 in das Lesegerät 17 eingeschoben, so ist das Meßgerät betriebsbereit. Es ist in Fig. 3 in der Meßbetriebsart dargestellt. Dabei ist die Sonde 5 in einem Stromkreis über einen Meßverstärker 20 an eine Polarographiespannungsquelle 21 angeschlossen. Die Polarographiespannungsquelle 21 läßt in der Sonde 5 zwischen Kathode 11 und Anode 13 durch den Elektrolyt einen Meßstrom fließen, dessen Höhe im Meßverstärker 20 bestimmt und über entsprechende Leitungsverbindungen (zur Verbesserung der Übersichtlichkeit sind in Fig. 3 die Leitungen nicht einzeln beziffert) auf dem Anzeigedisplay 16 anzeigt. Anstelle des Anzeigedisplays 16 kann der Meßwert des Stromes auch in nicht dargestellter Weise externen Einrichtungen zugeführt werden, wie z.B. einer zentralen Computeranlage oder sonstigen Auswerteinrichtungen, Meßschreibern od. dgl.

In der Leitung von der Steckkupplung 14 zum Meßverstärker 20 ist ein Umschalter 22 vorgesehen, der von einer Umschaltsteuereinrichtung 23 in zwei Schaltstellungen betätigbar ist. In Fig. 3 ist er in der Meßstellung dargestellt. Wird er in die andere Schaltstellung, die Regenerierstellung umgesteuert, so wird die Soude 5 unmittelbar an eine Regenerierspannungsquelle 24 angeschlossen, die, wie die in Fig. 3 in den Spannungsquellen 21 und 24 eingezeichneten Polaritäten anzeigen, Spannung umgekehrter Polarität an die Sonde 5 legt. Es wird dadurch im Elektrolyten zwischen Kathode 11 und Anode 13 ein Stromfluß in zur Meßrichtung umgekehrter Richtung erzwungen, mit dem in Umkehrung der bei der Messung ablaufenden chemischen Vorgänge der Elektrolyt regeneriert wird.

Die Umschaltung zwischen Regenerierbetrieb und Meßbetrieb kann beispielsweise in vorgegebenen Zeitabständen automatisch erfolgen oder kann von Hand über nicht dargestellte, auf der Außenseite des Meßgerätes 15 vorgesehene Schalter gesteuert werden.

In der dargestellten Ausführungsform des Meßgerätes 15 ist eine zentrale Steuereinrichtung 25 vorgesehen, die über Daten- und Steuerleitungen an die übrigen Funktionselemente des Meßgerätes angeschlossen ist. Bei diesen Leitungen ist jeweils mit einem Pfeil die Richtung angegeben, in der Daten- bzw. Steuersignale fließen. Die Steuereinrichtung 25 ist mit einer Sende- und einer Empfangsleitung an das Lesegerät 17 angeschlossen. Von diesem können eine große Vielzahl von Daten zur Verwertung bei der Messung empfangen werden. Solche Daten sind beispielsweise Herstellungsdaten der Sonde, wie beispielsweise exemplarspezifische Eichdaten. Diese Daten können vom Hersteller der Sonde auf dem Datenträger 18 abgespeichert werden. Diese sondenspezifischen Daten werden von dem Steuergerät 25 nach geeigneter Aulbereitung über eine Dalensendeleitung an den Meßverslärker 20 gegeben, damit dieser den Meßwert entsprechend korrigiert.

Ferner können auf dem Datenträger 18 patientenspezifische Daten (Name, Aller etc.) gespeichert sein, die beispielsweise auf Wunsch auf dem Anzeigedisplay 16 zur Anzeige gebracht werden können.

Es führt auch eine Datenempfangsleitung vom Meßverstärker 20 zum Steuergerät 25. Es können also laufend die Meßdaten auf dem Datenträger 18 gespeichert werden, beispielsweise um sie später von diesem abzurufen. Ferner kann das Steuergerät 25 z.B. die Meßzeiten der Sonde 5 laufend auf dem Datenträger 18 abspeichern, um jederzeit das Alter der Sonde, also die zulässige Betriebslebensdauer ermitteln zu können.

Da der Datenträger 18 z.B. über die Sicherheitskette 19 oder auf andere Weise unverlierbar mit der Sonde 5 gekoppelt ist, läßt sich auf diese Weise die Sonde mit unterschiedlichen Meßgeräten verwenden, die stets auf den Datenvorrat des Datenträgers 18 zurückgreifen können. Es kann so gearbeitet werden, als wäre die Sonde nie vom Meßgerät getrennt gewesen.

Im dargestellten Ausführungsbeispiel steuert die Steuereinrichtung 25 über entsprechende Steuerleitungen auch die Umschaltsteuereinrichlung 23 zum Umschalten zwischen Meßbetrieb und Regenerierbetrieb und steuert auch die Polarografierspannungsquelle 21 und die Regenerierspannungsquelle 24, um diese je nach Betriebsart ein- und auszuschalten.

Die Steuereinrichtung 25 kann auch die von den Spannungsquellen, also der Polarographierspannungsquelle 21 und der Regenerierspannungsquelle 24 gelieferten Spannungen steuern oder gegebenenfalls im Falle der Regenerierspannungsquelle 24 deren Ausgangsstrom steuern. In einer alternativen Ausbildung kann der von der Regenerierspannungsquelle 24 gelieferte Strom bei geeignetem Schaltungsaufbau durch den Meßverslärker 20 fließen, damit dieser den Regenerierbetrieb überwachen kann.

Die Steuereinrichtung 25 erhält während des Meßbetriebes laufend die Höhe des Meßstromes vom Meßverstärker 20 und erhält ferner ein Zeitsignal von einem Timer 26. Daraus kann sie den Elektrolytverbrauch errechnen, beispielsweise als Integral des Meßstromes über der Zeit. Aus vorgegebenen Grenzwerten des Elektrolytverbrauches, die sie beispielsweise von dem Datenträger 18 liest, kann sie den Zeitpunkt bestimmen, zu dem der Elektrolyt erschöpft ist, und kann dann auf Regenerierbetrieb umschalten. Sie kann dabei wiederum auf ähnliche Weise Dauer und Stromstärke bestimmen und nach ausreichend regenerierter Elektrolytmenge zurückschalten auf Meßbetrieb.

Wird während des Meßbetriebes oder während des Regenerierbetriebes aus medizinischen oder organisatorischen Gründen eine Abkopplung der Sonde 5 vom Meßgerät 15 erforderlich, z.B. bei einer Verlegung des Patienten in ein anderes Bett, so ist der aktuelle Zustand auf dem Datenträger 18 gespeichert, und es kann die Sonde 5 nach abgeschlossener Verlegung des Patienten wieder mit dem Meßgerät oder einem anderen Meßgerät gekoppelt werden, und dieses kann mit den Daten auf dem Datenträger 18 genau dort weitermachen, wo vor der Betriebsunterbrechung aufgehört wurde.

Das Steuergerät 25 kann, wie erwähnt, Daten laufend mit dem Lesegerät 17 auf den Datenträger 18 abspeichern oder auch diese selbst zwischenspeichern und nur auf Bedarf, wenn eine Abkopplung der Sonde 5 bevorsteht, abspeichern. Ebenso kann es auch die zu lesenden Daten entweder bei Bedarf vom Datenträger 18 einzeln holen oder beim Anschluß der Sonde 5 komplett von diesem herunterspielen und in einen eigenen Zwischenspeicher laden.

## Patentansprüche

1. Meßvorrichtung zur Bestimmung des Sauerstoffpartialdruckes im menschlichen Gehirngewebe (3), mit einer Sonde (5), die mit einem dünnen flexiblen Schlauch (7) aus gewebeverträglichem Kunststoffmaterial in ihrem Meßbereich einen mit Elektrolyt (8) gefüllten Raum umschließt, in dem eine polarographische Kathode (11) mit begrenzter Oberfläche und eine Anode (13) angeordnet sind, wobei der Schlauch (7) wenigstens im Meßbereich sauerstoffdurchlässig ausgebildet ist und wobei diese Elektroden (11, 13) an dem vom Meßbereich entfernt liegenden Ende der Sonde (5) an ein Meßgerät (15) angeschlossen sind, das an die Elektroden (11, 13) eine Spannungsquelle (21) zur Erzeugung der Polarographiespannung anlegt und mit einem Meßverstärker (20) den Meßstrom bestimmt, wobei eine Regeneriereinrichtung (24) vorgesehen ist, die nach Umschaltung der Elektrodenanschlüsse diese in umgekehrter Polarität versorgt, **dadurch gekennzeichnet, daß** ein der Sonde (5) zugeordneter Datenträger (18) vorgesehen ist und daß das Meßgerät (15) dazu ausgebildet ist, die Betriebsdaten des Polarographiebetriebes dauernd oder bei Abkopplung einer Sonde (5) auf den Datenträger (18) zu schreiben und diese Daten bei Anschluß einer Sonde (5) von deren Datenträger (18) zu lesen.

2. Meßvorrichtung nach Anspruch 1, **dadurch gekennzeichnet, daß** als Elektrolyt (8) eine wässerige Lösung von Natriumazetat, Natriumphosphat, Essigsäure und Natriumdihydrogenphosphat vorgesehen ist.

3. Meßvorrichtung nach Anspruch 1, **dadurch gekennzeichnet, daß** die Anode (13) eine Silberoberfläche aufweist.

4. Meßvorrichtung nach Anspruch 1, **dadurch gekennzeichnet, daß** das Meßgerät (15) dazu ausgebildet ist, die Meßzeit und den Meßstrom im Polarographiebetrieb einer Sonde (5) zu ermitteln und danach Zeitpunkt und Dauer sowie Stromstärke des Regenerierbetriebes zu berechnen.

## Claims

1. Measuring device for determining the oxygen partial pressure in human brain tissue (3) including a probe (5), which, in its measuring region, surrounds with a thin flexible hose (7) of tissue-compatible plastic material a space, which is filled with electrolyte (8) and in which are arranged a polarographic cathode (11) with a limited surface and an anode (13), the hose (7) being oxygen-permeable, at least in the measuring region, and these electrodes being connected at the end of the probe (5) remote from the measuring region to a measuring device (15), which applies to the electrodes (11, 13) a voltage source (21) for producing the polarography voltage and determines the measuring current with a measurement amplifier (20), a regenerating device (24) being provided which, after switching over the electrode connections, supplies them with reversed polarity, **characterised in that** a data carrier is provided associated with the probe (5) and that the measuring device (15) is constructed to write the operational data of the polarography operation onto the data carrier (18) continuously or when decoupling a probe (5) and to read this data, when connecting a probe (5), from its data carrier (18).

2. Measuring device as claimed in Claim 1, **characterised in that** an aqueous solution of sodium acetate, sodium phosphate, acetic acid and sodium dihydrogen phosphate is provided as the electrolyte.

3. Measuring device as claimed in Claim 1, **characterised in that** the anode (13) has a silver surface.

4. Measuring device as claimed in Claim 1, **characterised in that** the measuring device (15) is constructed to determine the measuring time and the measuring current in polarographic operation of a probe (5) and to calculate the time and duration and current intensity of the regeneration operation therefrom.

## Revendications

1. Dispositif de mesure permettant de déterminer la pression partielle d'oxygène dans le tissu cérébral humain (3) équipé d'une sonde (5) munie d'un fin tuyau flexible (7) en matériau synthétique compatible avec les tissus et enveloppant, dans la section destinée à la mesure, un espace rempli d'électrolyte (8) dans lequel sont agencées une cathode polarographique (11) de surface limitée et une anode (13), le tuyau (7) étant, au moins dans la section de mesure, de type perméable à l'oxygène, et les électrodes (11, 13) étant, à l'extrémité de la sonde distante de la section de mesure, branchées sur un appareil de mesure (15) qui met les électrodes (11, 13) en contact avec une source de tension (21) afin de produire la tension de polarographie et détermine au moyen d'un amplificateur de mesure (20) le courant mesuré, un dispositif de régénération (24) étant prévu pour appliquer aux électrodes, après inversion de leurs connexions, une polarité inverse, **caractérisé en ce qu**'est prévu un support de données (18) affecté à la sonde (5), et en ce que l'appareil de mesure (15) est conçu pour inscrire soit en permanence, soit au moment du désaccouplement d'une sonde (5), les données polarographiques sur le support de données (18) et, lors du branchement d'une sonde (5), lire ces données de son support de données (18).

2. Dispositif de mesure selon la revendication 1, **caractérisé en ce que** l'électrolyte (8) prévu est une solution aqueuse d'acétate de sodium, de phosphate de sodium, d'acide acétique et de dihydrophosphate de sodium.

3. Dispositif de mesure selon la revendication 1, **caractérisé en ce que** l'anode (139 présente une surface d'argent.

4. Dispositif de mesure selon la revendication 1, **caractérisé en ce que** l'appareil de mesure (15) est conçu de façon à pouvoir déterminer la durée de mesure et le courant de mesure d'une sonde (5) en service polarographique, puis de calculer le moment du déclenchement, la durée et l'intensité du courant du service de régénération.
